(19) **Europäisches Patentamt**

**European Patent Office**

**Office européen des brevets**

(11) **EP 1 247 795 B1**

(12) **FASCICULE DE BREVET EUROPEEN**

(45) Date de publication et mention
de la délivrance du brevet:
**10.08.2005 Bulletin 2005/32**

(51) Int Cl.⁷: **C07C 67/58**, C07C 69/54

(21) Numéro de dépôt: **02290719.0**

(22) Date de dépôt: **21.03.2002**

(54) **Procédé de fabrication d'acrylates de 2-éthylhexyle**

Verfahren zur Herstellung von 2-Ethylhexylacrylat

Process for the production of 2-ethylhexyl acrylate

(84) Etats contractants désignés:
**AT BE CH CY DE DK ES FI FR GB GR IE IT LI LU
MC NL PT SE TR**

(30) Priorité: **02.04.2001 FR 0104442**

(43) Date de publication de la demande:
**09.10.2002 Bulletin 2002/41**

(73) Titulaire: **ARKEMA
92800 Puteaux (FR)**

(72) Inventeurs:
• **Bessalem, Jacqueline
57500 Saint-Avold (FR)**

• **Fauconet, Michel
57730 Valmont (FR)**
• **Lepizzera, Stéphane
57500 Saint-Avold (FR)**

(74) Mandataire: **Rieux, Michel
ARKEMA
Département Propriété Industrielle
4-8, cours Michelet,
La Défense 10
92091 Paris la Défense Cedex (FR)**

(56) Documents cités:
**EP-A- 0 463 434          EP-A- 0 609 127
US-A- 5 606 102**

**Description**

La présente invention concerne un procédé perfectionné de fabrication d'acrylate de 2-éthylhexyle par une estérification directe de l'acide acrylique par le 2-éthylhexanol, cette réaction étant catalysée par de l'acide sulfurique.

Dans ce procédé industriel, pour déplacer l'équilibre réactionnel, il n'est pas rajouté de solvant entraînant azéotropiquement l'eau de réaction, mais cette fonction est assurée par un excès de l'alcool estérifiant (en l'occurrence, le 2-éthylhexanol), qui présente la particularité de constituer un azéotrope avec l'eau.

À l'issue de l'étape réactionnelle réalisée en discontinu, la quasi-totalité de l'acide sulfurique a été transformée en sulfate acide de 2-éthylhexyle (Et2HexSO$_4$H), selon la réaction suivante d'estérification de l'acide sulfurique par le 2-éthylhexanol :

$$2\text{-EtHexOH} + H_2SO_4 \rightarrow 2\text{-EtHexSO}_4H + H_2O$$

Par conséquent, le mélange réactionnel, en fin de réaction, comprend de l'acrylate de 2-éthylhexyle, du 2-éthylhexanol, de l'acide acrylique, du sulfate acide de 2-éthylhexyle, des traces d'acide sulfurique, et les stabilisants classiquement utilisés dans la réaction.

Dans le procédé traditionnel, on fait suivre la réaction d'estérification par des étapes de purification, généralement effectuées en continu :

- on neutralise les espèces acides contenues dans le mélange brut réactionnel, par addition à celui-ci d'une solution aqueuse de base (soude) ; pendant cette étape, l'acide acrylique est neutralisé en acrylate de sodium, le sulfate acide de 2-éthylhexyle, en sulfate neutre de 2-éthylhexyle Et2HexSO$_4$Na, et les traces d'acide sulfurique, en sulfate de sodium Na$_2$SO$_4$, tous ces sels passant dans la phase aqueuse ;
- on lave à l'eau, dans une colonne d'extraction, la phase organique séparée, issue de la neutralisation, afin d'éliminer les traces d'impuretés, de soude et de sels, puis l'on envoie l'acrylate de 2-éthylhexyle lavé sur une première colonne à distiller, permettant d'éliminer en tête les produits légers, lesquels sont recyclés à la réaction d'estérification ; on envoie ensuite l'ester étêté sur une deuxième colonne, d'où ils sort en tête, purifié des produits lourds ;
- on récupère le 2-éthylhexanol présent :

  - dans la phase aqueuse séparée, issue de la neutralisation, laquelle contient essentiellement du sulfate neutre de 2-éthylhexyle, du 2-éthylhexanol, de l'acrylate de sodium et des traces de sulfate de sodium ;
  - dans la phase aqueuse issue de la réaction d'estérification proprement dite ainsi que

  - dans la phase aqueuse issue du lavage dans la colonne d'extraction de la phase organique,

cette récupération du 2-éthylhexanol étant réalisée dans une colonne de distillation, alimentée en tête par les eaux à traiter, dans laquelle le 2-éthylhexanol est entraîné en tête et peut être recyclé à la réaction d'estérification, tandis que le pied de colonne constitue les eaux usées, débarrassées du 2-éthylhexanol, qui seront rejetées à la station de traitement biologique.

Les eaux ainsi rejetées contenant une forte pollution organique (mesurée par la demande chimique en oxygène (DCO)) à cause de la présence de sulfate neutre de 2-éthylhexyle et d'acrylate de sodium, un procédé perfectionné de fabrication de l'acrylate de 2-éthylhexyle a été mis au point, lequel fait l'objet de la demande de brevet européen EP-A-609 127.

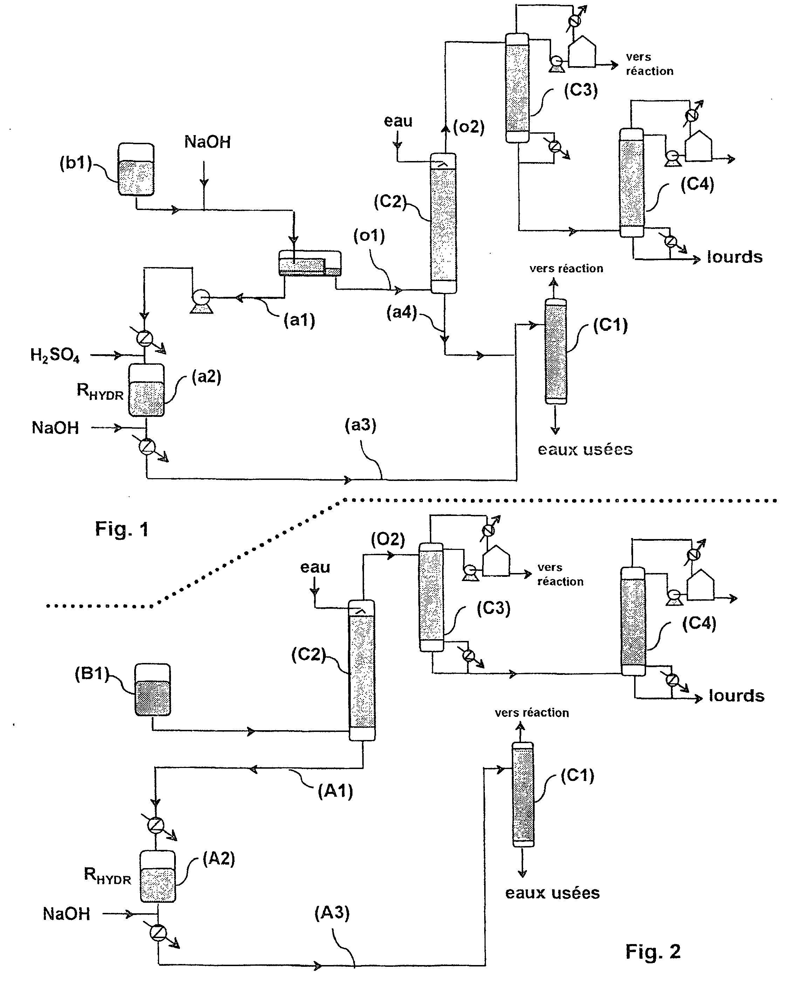

Comme on peut le décrire avec référence à la Figure 1 du dessin annexé, conformément à ce procédé connu, le mélange réactionnel (b1) est, comme dans le procédé traditionnel, neutralisé par une solution de soude, et, à l'issue de cette neutralisation, le mélange décante en deux phases : une phase aqueuse (a1) et une phase organique (o1).

Après addition d'acide sulfurique dans (a1) pour obtenir un rapport molaire du nombre d'équivalents H$^+$ en excès au nombre de moles de sulfate acide de 2-éthylhexyle au moins égal à 1,5, le nouveau mélange (a2) est introduit dans un réacteur d'hydrolyse R$_{HYD}$ et porté à une température de l'ordre de 70-200°C pendant un temps de l'ordre de 1-5 heures. Le mélange après réaction est neutralisé jusqu'à pH 8 par NaOH à 8 %. La phase (a3) résultante est adressée en continu sur une colonne de distillation C1 pour récupération du 2-éthylhexanol en tête et concentration des eaux épuisées destinées au traitement biologique en pied.

Après une nouvelle neutralisation par NaOH, la phase organique séparée (o1) est lavée à l'eau dans une colonne d'extraction C2 afin d'éliminer les traces d'impuretés, de soude et de sels. La phase aqueuse (a4) issue du pied de la colonne d'extraction C2 est adressée à une colonne de distillation C1.

L'acrylate de 2-éthylhexyle lavé, obtenu en tête de la colonne C2, est adressé à une première colonne à distiller C3 permettant d'éliminer en tête les produits légers constitués par essentiellement du 2-éthylhexanol, lesquels sont recyclés à la réaction d'estérification. L'acrylate de 2-éthylhexyle étêté, obtenu en pied, est envoyé sur la deuxième colonne de distillation C4, d'où il sort en tête, purifié des produits lourds.

Lors de la mise en oeuvre de ce procédé de fabrication de l'acrylate de 2-éthylhexyle, il apparaît qu'il se forme une émulsion dense à l'interphase lors de la décantation qui se fait suite à l'étape de neutralisation précitée. Cette émulsion, qui pourrait être due à des polymères d'acrylate de 2-éthylhexyle se développant à l'interphase, perturbe le train de distillation en aval et abaisse les performances environnementales de l'atelier de fabrication.

L'étape de neutralisation du brut réactionnel génère l'essentiel de la DCO de l'atelier due à l'acrylate de sodium qui a pour origine, d'une part, la neutralisation de l'acide acrylique non transformé et, d'autre part, la saponification de l'acrylate de 2-éthylhexyle.

La Société déposante a découvert que ces problèmes pouvaient être résolus sans traitement chimique, par la suppression de la neutralisation par NaOH des phases (b1) et (o1), le sulfate acide de 2-éthylhexyle étant éliminé par lavage à l'eau et décantation dans la colonne C2, l'acide acrylique résiduel, non extrait en phase aqueuse comme c'était le cas dans le procédé connu selon EP-A-609 127, étant éliminé en tête de la colonne d'étêtage C3.

La présente invention a donc pour objet un procédé de fabrication d'acrylate de 2-éthylhexyle par estérification directe de l'acide acrylique par le 2-éthylhexanol, en présence d'au moins un stabilisant pour l'acide acrylique, ladite estérification étant catalysée par l'acide sulfurique, le mélange réactionnel brut (B1) obtenu comprenant de l'acrylate de 2-éthylhexyle, du 2-éthylhexanol, de l'acide acrylique, du sulfate acide de 2-éthylhexyle, des traces d'acide sulfurique et les impuretés habituelles, caractérisé par le fait que l'on conduit un lavage à l'eau du mélange réactionnel brut (B1)généralement à une température de 20-50°C dans une colonne d'extraction C2 permettant d'obtenir, en tête, une phase organique (O2) et d'éliminer, en pied, une phase aqueuse (A1),

- la phase organique (O2) étant adressée à une colonne d'étêtage C3 permettant d'obtenir en tête l'acide acrylique et le 2-éthylhexanol contenus dans la phase (O2), lesquels sont recyclés à la réaction d'estérification, l'acrylate de 2-éthylhexyle étêté étant adressé à une deuxième colonne de distillation C4 d'où il sort, en tête, purifié des produits lourds ; et
- la phase aqueuse (A1) étant adressée à une étape d'hydrolyse du sulfate acide de 2-éthylhexyle présent dans ladite phase aqueuse (A1), l'hydrolyse étant conduite à une température comprise entre 70 et 200°C de manière à former dans ladite phase du 2-éthylhexanol et de l'acide sulfurique, les espèces acides résultant de ladite hydrolyse étant neutralisées par introduction d'une base dans le milieu, et la phase aqueuse résultante (A3) étant adressée à une étape de récupération du 2-éthylhexanol dans une colonne de distillation C1, ledit alcool étant entraîné en tête de ladite colonne C1.

Conformément à un mode de réalisation particulier du procédé selon l'invention :

- on effectue le lavage à l'eau du mélange réactionnel brut (B1) avec utilisation de 5 à 50 % en poids d'eau par rapport à la charge de mélange réactionnel brut (B1)
- on conduit l'hydrolyse à une température comprise entre 70°C et 200°C.

Pour mieux illustrer l'objet de la présente invention, on va en décrire ci-après un exemple de mise en oeuvre (Exemple 1), avec référence à la Figure 2 du dessin annexé, qui représente le schéma du procédé selon l'invention. L'Exemple comparatif 2 est présenté, avec référence à la Figure 1 précitée, laquelle illustre le procédé connu. Dans les exemples, les parties et pourcentages sont en poids.

Le cheminement des différents flux illustrés sur les Figures 1 et 2 a été décrit ci-dessus.

<u>EXEMPLE 1 (de l'invention)</u>

<u>A - SYNTHÈSE DU MÉLANGE BRUT RÉACTIONNEL</u>

On conduit, en discontinu, dans un réacteur agité, à une température de 90°C, sous pression réduite, une estérification d'acide acrylique (466,3 parties) par le éthylhexanol (520 parties), en présence d'acide sulfurique à 94% (9,76 parties) comme catalyseur, et en présence de phénothiazine comme inhibiteur de polymérisation (0,7 partie). L'équilibre de la réaction d'estérification est déplacé vers la formation de l'ester attendu acrylate de 2-éthylhexyle par distillation de l'eau générée, sous forme d'un mélange azéotropique avec le 2-éthylhexanol.

La composition du brut réactionnel ($B_1$) est la suivante :

| | |
|---|---|
| Acrylate de 2-éthylhexyle | 91 % |
| 2-Ethylhexanol | 4,4 % |
| Acide acrylique | 0,5 % |
| Sulfate acide de 2-éthylhexyle | 2,5 % |
| Eau | 0,5 % |
| Autres impuretés | 1,1 % |

B - <u>EXTRACTION DU CATALYSEUR PAR TROIS LAVAGES À L'EAU SIMULATION DE LA COLONNE C2</u>

A 460 g de brut réactionnel (B1) contenant le catalyseur (0,12 éq $H^+$/kg d'acidité forte - sulfate acide de 2-éthylhexyle), on ajoute 46 g d'eau et on agite à température ambiante pendant 10 minutes. Après décantation, on récupère 452 g de phase organique (O1) contenant 0,011 éq $H^+$/kg d'acidité forte.

Le rendement d'extraction du catalyseur est de 91 %.

Cette opération est renouvelée 2 fois (traitement de la phase organique (O1) par 10% d'eau en poids), de façon à simuler le fonctionnement de la colonne C2.

En sortie de colonne C2, le brut réactionnel lavé (O2) contient $6 \times 10^{-5}$ $H^+$/kg. Le rendement global d'extraction du catalyseur est de 99,5 %.

C - PURIFICATION DU BRUT (O2)

(O2) est envoyé vers la colonne C3 pour sortir, en tête :

-  l'acide acrylique et le 2-éthylhexanol essentiellement ;
  et, en pied :

-  l'acrylate de 2-éthylhexyle renfermant le stabilisant et les produits lourds (essentiellement ester du dimère de l'acide acrylique et l'octoxypropionate d'octyle).

Le pied de cette colonne C3 est envoyé vers la colonne C4 pour sortir en tête de C4 l'acrylate de 2-éthylhexyle pur (pureté [3] 99,7 %).

H - HYDROLYSE DES PHASES AQUEUSES (A1) ET (A5)

La phase aqueuse (A1) issue de la colonne C2 est envoyée vers l'hydrolyseur $R_{HYD}$ pour décomposer le sulfate de 2-éthylhexyle en $H_2SO_4$ et 2-éthylhexanol (T = 130°C ; durée = 2 heures ; Pression : 2-3 bars).

Le flux sortant de l'hydrolyseur $R_{HYD}$ est neutralisé par NaOH et envoyé vers la colonne C1 pour récupérer, en tête de colonne, le 2-éthylhexanol.

EXEMPLE 2 (comparatif)

On a conduit le procédé comme décrit avec référence à la Figure 1, ce procédé mettant en oeuvre la neutralisation du brut réactionnel (b1) ayant la même composition que (B1). Ce procédé a conduit à une émulsion dense lors de l'étape de neutralisation et à la perte de l'acide acrylique.

**Revendications**

**1.**  Procédé de fabrication d'acrylate de 2-éthylhexyle par estérification directe de l'acide acrylique par le 2-éthylhexanol, en présence d'au moins un stabilisant pour l'acide acrylique, ladite estérification étant catalysée par l'acide sulfurique, le mélange réactionnel brut (B1) obtenu comprenant de l'acrylate de 2-éthylhexyle, du 2-éthylhexanol, de l'acide acrylique, du sulfate acide de 2-éthylhexyle, des traces d'acide sulfurique et les impuretés habituelles, **caractérisé par le fait que** l'on conduit un lavage à l'eau du mélange réactionnel brut (B1) à une température de 20-50°C dans une colonne d'extraction C2 permettant d'obtenir, en tête, une phase organique (O2) et d'éliminer, en pied, une phase aqueuse (A1),

-  la phase organique (O2) étant adressée à une colonne d'étêtage C3 permettant d'obtenir en tête l'acide acrylique et le 2-éthylhexanol contenus dans la phase (O2), lesquels sont recyclés à la réaction d'estérification, l'acrylate de 2-éthylhexyle étêté étant adressé à une deuxième colonne de distillation C4 d'où il sort, en tête, purifié des produits lourds ; et

-  la phase aqueuse (A1) étant adressée à une étape d'hydrolyse du sulfate acide de 2-éthylhexyle présent dans ladite phase aqueuse (A1), l'hydrolyse étant conduite de manière à former dans ladite phase du 2-éthylhexanol et de l'acide sulfurique, les espèces acides résultant de ladite hydrolyse étant neutralisées par introduction d'une base dans le milieu, et la phase aqueuse résultante (A3) étant adressée à une étape de récupération du 2-éthylhexanol dans une colonne de distillation C1, ledit alcool étant entraîné en tête de ladite colonne C1.

**2.**  Procédé selon la revendication 1, **caractérisé par le fait que** l'on effectue le lavage à l'eau du mélange réactionnel brut (B1) avec utilisation de 5 à 50 % en poids d'eau par rapport à la charge de mélange réactionnel brut (B1).

**3.**  Procédé selon l'une des revendications 1 et 2, **caractérisé par le fait que** l'on conduit l'hydrolyse à une température comprise entre 70 et 200°C.

**Patentansprüche**

**1.**  Verfahren zur Herstellung von 2-Ethylhexylacrylat durch direkte Veresterung von Acrylsäure mit 2-Ethylhexanol in Gegenwart mindestens eines Acrylsäure-Stabilisators unter Schwefelsäure-Katalyse, wobei das rohe Reaktionsgemisch (B1) 2-Ethylhexylacrylat, 2-Ethylhexanol, Acrylsäure, 2-Ethylhexylhydrogensulfat, Spuren von Schwefelsäure und übliche Verunreinigungen enthält, **dadurch gekennzeichnet, daß** man das rohe Reaktionsgemisch (B1) bei einer Temperatur von 20-50°C in einer Extraktionssäule C2 mit Wasser wäscht, in welcher man am Kopf eine organische Phase (O2) erhält und im Sumpf eine wäßrige Phase (A1) abzieht,

-  wobei man die organische Phase (O2) einer Toppsäule C3 zuführt, in welcher man am Kopf die in der Phase (O2) enthaltene Acrylsäure und das in der Phase (O2) enthaltene 2-Ethylhexanol erhält, welche in die Veresterungsreaktion zurückgeführt werden, und das getoppte 2-Ethylhexylacrylat einer zweiten Destillationssäule C4 zuführt, aus welcher es am Kopf von schweren Produkten gereinigt austritt; und

- wobei man die wäßrige Phase (A1) einem Schritt der Hydrolyse des in der wäßrigen Phase (A1) vorliegenden 2-Ethylhexylhydrogensulfats zuführt, wobei die Hydrolyse so durchgeführt wird, daß sich in der Phase 2-Ethylhexanol und Schwefelsäure bilden, die bei der Hydrolyse anfallenden sauren Spezies durch Eintragen einer Base in das Medium neutralisiert werden und die erhaltene wäßrige Phase (A3) einem Schritt der Rückgewinnung von 2-Ethylhexanol in einer Destillationssäule C1 zugeführt wird, wobei der Alkohol zum Kopf der Säule C1 geführt wird.

2. Verfahren nach Anspruch 1, **dadurch gekennzeichnet, daß** man die Wasserwäsche des rohen Reaktionsgemischs (B1) mit 5 bis 50 Gew.-% Wasser, bezogen auf die Charge des rohen Reaktionsgemischs (B1), durchführt.

3. Verfahren nach Anspruch 1 oder 2, **dadurch gekennzeichnet, daß** man die Hydrolyse bei einer Temperatur zwischen 70 und 200°C durchführt.

## Claims

1. Process for the manufacture of 2-ethylhexyl acrylate by direct esterification of acrylic acid with 2-ethylhexanol in the presence of at least one stabilizer for acrylic acid, the said esterification being catalysed by sulphuric acid, the crude reaction mixture (B1) obtained comprising 2-ethylhexyl acrylate, 2-ethylhexanol, acrylic acid, 2-ethylhexyl hydrogensulphate, traces of sulphuric acid and the usual impurities, **characterized in that** washing of the crude reaction mixture (B1) with water is carried out at a temperature of 20-50°C in an extraction column C2 which makes it possible to obtain, at the top, an organic phase (O2) and to remove, at the bottom, an aqueous phase (A1),

   - the organic phase (O2) being sent to a topping column C3 which makes it possible to obtain, at the top, the acrylic acid and the 2-ethylhexanol present in the phase (O2), which products are recycled to the esterification reaction, the topped 2-ethylhexyl acrylate being sent to a second distillation column C4, from where it emerges at the top, purified from the heavy products; and
   - the aqueous phase (A1) being sent to a stage of hydrolysis of the 2-ethylhexyl hydrogensulphate present in the said aqueous phase (A1), the hydrolysis being carried out so as to form, in the said phase, 2-ethylhexanol and sulphuric acid, the acidic entities resulting from the said hydrolysis being neutralized by introduction of a base into the medium, and the resulting aqueous phase (A3) being sent to a stage of recovery of the 2-ethylhexanol in a distillation column C1, the said alcohol being entrained into the top of the said column C1.

2. Process according to Claim 1, **characterized in that** the washing of the crude reaction mixture (B1) with water is carried out with the use of 5 to 50% by weight of water with respect to the charge of crude reaction mixture (B1).

3. Process according to either of Claims 1 and 2, **characterized in that** the hydrolysis is carried out at a temperature of between 70 and 200°C.

Fig. 1

Fig. 2